# EUROPÄISCHE PATENTANMELDUNG

(11) **EP 1 106 689 A2**
(43) Veröffentlichungstag der Anmeldung: **13.06.2001**
(21) Anmeldenummer: 00125300.4
(22) Anmeldetag: 28.11.2000
(51) Int. Cl.: C12N 15/12, C07K 14/705, C07K 16/28, G01N 33/50, C12Q 1/68, A01K 67/033, C07K 14/435

(54) **Nukleinsäuren, die für Acetylcholinrezeptor-beta-Untereinheiten für Insekten kodieren**

(30) Priorität: 10.12.1999 DE 19959582
(71) Anmelder: BAYER AG, 51368 Leverkusen (DE)
(72) Erfinder: Adamczewski, Martin, Dr., 51067 Köln (DE); Methfessel, Christoph, Dr., 42327 Wuppertal (DE); Schulte, Thomas, Dr., 51061 Köln (DE)

(57) **Zusammenfassung**

Die Erfindung betrifft Nukleinsäuren, die für Acetylcholinrezeptor-β-Untereinheiten von Insekten kodieren, sowie Polypeptide, welche die biologische Funktion solcher Acetylcholinrezeptor-β-Untereinheiten ausüben, und insbesondere deren Verwendung zum Auffinden von Wirkstoffen für den Pflanzenschutz.

SEQUENZPROTOKOLL

<110> Bayer Aktiengesellschaft

<120> Nukleinsäuren, die für neue
      Acetylcholinrezeptor-beta-Untereinheiten von Insekten
      kodieren

<130> Le A 34 147

<140>
<141>

<150> DE 199 59 582.8
<151> 1999-12-10

<160> 4

<170> PatentIn Ver. 2.1

<210> 1
<211> 1539
<212> DNA
<213> Drosophila melanogaster

<220>
<221> CDS
<222> (43)..(1365)

<400> 1
attcggcacg agggtacatc cgaaacaaag gcgcgctgaa ca atg acg acg act     54
                                              Met Thr Thr Thr
                                                1

ccc aag ata aag gca cca gtt tcc ggt cct gga ctg cca cta ctg ctg    102
Pro Lys Ile Lys Ala Pro Val Ser Gly Pro Gly Leu Pro Leu Leu Leu
  5                  10                  15                  20

caa atg cta atg ggg atg ctt ctt atg ggg ctg act tcc gtg cca ggc    150
Gln Met Leu Met Gly Met Leu Leu Met Gly Leu Thr Ser Val Pro Gly
                 25                  30                  35

gcc act gcc acc gcg gac ccc aag aac gcc aat gtc aag gcc ctg gat    198
Ala Thr Ala Thr Ala Asp Pro Lys Asn Ala Asn Val Lys Ala Leu Asp
             40                  45                  50

cgc ctc cac gcc ggc ctg ttc acg aac tac gac agc gat gtg cag ccg    246
Arg Leu His Ala Gly Leu Phe Thr Asn Tyr Asp Ser Asp Val Gln Pro

        55                  60                  65

gtg ttc caa gga acc ccc acg aac gtg tcc ctg gaa atg gtg gtc acc 294
Val Phe Gln Gly Thr Pro Thr Asn Val Ser Leu Glu Met Val Val Thr
    70                  75                  80

tac ata gac atc gac gag ttg aac ggc aag ctg acc acc cac tgc tgg 342
Tyr Ile Asp Ile Asp Glu Leu Asn Gly Lys Leu Thr Thr His Cys Trp
85                  90                  95                  100

ctg aat ctc cga tgg aga gac gag gag cgc gtg tgg caa ccg tca caa 390
Leu Asn Leu Arg Trp Arg Asp Glu Glu Arg Val Trp Gln Pro Ser Gln
                105                 110                 115

tat gac aac atc acg cag atc act ttg aag tcc agc gag gtc tgg acc 438
Tyr Asp Asn Ile Thr Gln Ile Thr Leu Lys Ser Ser Glu Val Trp Thr
            120                 125                 130

ccc caa atc aca ctc ttc aac ggc gac gaa ggt ggc ctg atg gcc gaa 486
Pro Gln Ile Thr Leu Phe Asn Gly Asp Glu Gly Gly Leu Met Ala Glu
        135                 140                 145

acc cag gtg acc ctc agc cac gat ggc cac ttc cgg tgg atg cct cca 534
Thr Gln Val Thr Leu Ser His Asp Gly His Phe Arg Trp Met Pro Pro
    150                 155                 160

gcc gtg tac acg gcc tac tgc gaa ctc aac atg ctc aac tgg ccc cac 582
Ala Val Tyr Thr Ala Tyr Cys Glu Leu Asn Met Leu Asn Trp Pro His
165                 170                 175                 180

gac aag cag agc tgc aag ttg aag atc ggc tcc tgg ggc ctg aag gtc 630
Asp Lys Gln Ser Cys Lys Leu Lys Ile Gly Ser Trp Gly Leu Lys Val
                185                 190                 195

gtc ctg ccg gag aac ggc acg gcg aga gga gag tcc ctt gac cac gac 678
Val Leu Pro Glu Asn Gly Thr Ala Arg Gly Glu Ser Leu Asp His Asp
            200                 205                 210

gac ctg gtt cag tca ccg gag tgg gaa atc gtg gac tcg cga gcc cac 726
Asp Leu Val Gln Ser Pro Glu Trp Glu Ile Val Asp Ser Arg Ala His
        215                 220                 225

ttt gtc agt cag gac tac tac ggc tac atg gag tac act ctg acg gct 774
Phe Val Ser Gln Asp Tyr Tyr Gly Tyr Met Glu Tyr Thr Leu Thr Ala
    230                 235                 240

cag cgg cgc tcc tcc atg tac acg gcc gtc atc tac aca ccc gcg tcc 822
Gln Arg Arg Ser Ser Met Tyr Thr Ala Val Ile Tyr Thr Pro Ala Ser

245                 250                 255                 260

tgc atc gtc atc ctg gcc ctc tca gcc ttc tgg ctg cct ccc cac atg     870
Cys Ile Val Ile Leu Ala Leu Ser Ala Phe Trp Leu Pro Pro His Met
                265                 270                 275

ggc ggc gag aag atc atg atc aac ggc ctg ctc atc atc gtg atc gcc     918
Gly Gly Glu Lys Ile Met Ile Asn Gly Leu Leu Ile Ile Val Ile Ala
            280                 285                 290

gcc ttc ctc atg tac ttc gcc cag ctc ctg cca gtg ctg tcc aac aat     966
Ala Phe Leu Met Tyr Phe Ala Gln Leu Leu Pro Val Leu Ser Asn Asn
        295                 300                 305

act cca ctt gtg gta atc ttc tac agc acc agc ctg ctg tat ctg agc     1014
Thr Pro Leu Val Val Ile Phe Tyr Ser Thr Ser Leu Leu Tyr Leu Ser
    310                 315                 320

gtc tcc acc atc gtc gag gtt cta gtt ctg tac ctg gcc aca ggc aag     1062
Val Ser Thr Ile Val Glu Val Leu Val Leu Tyr Leu Ala Thr Gly Lys
325                 330                 335                 340

cac aag agg cgc ctg ccg gag gcg ctg aga aag ctg ctg cac ggg cac     1110
His Lys Arg Arg Leu Pro Glu Ala Leu Arg Lys Leu Leu His Gly His
                345                 350                 355

ctg ggc acg tgg ctg ctg ctc tcg gtg ttc agc acc act ggc gag tcg     1158
Leu Gly Thr Trp Leu Leu Leu Ser Val Phe Ser Thr Thr Gly Glu Ser
            360                 365                 370

cag gcg gag aag acc aaa gag atg gac gag cac ccg tac gag gag gcg     1206
Gln Ala Glu Lys Thr Lys Glu Met Asp Glu His Pro Tyr Glu Glu Ala
        375                 380                 385

gac gag cag gag tcc agt ccg ctg ggc atc aac cac acc gag gtg ccg     1254
Asp Glu Gln Glu Ser Ser Pro Leu Gly Ile Asn His Thr Glu Val Pro
    390                 395                 400

ggc gcc aag gcc aac cag ttc gac tgg gcg ctg ctg gcc acc gcc gtg     1302
Gly Ala Lys Ala Asn Gln Phe Asp Trp Ala Leu Leu Ala Thr Ala Val
405                 410                 415                 420

gac cgc att tcc ttc gtt tcc ttc agc ctg gcc ttc ctc att ctg gcc     1350
Asp Arg Ile Ser Phe Val Ser Phe Ser Leu Ala Phe Leu Ile Leu Ala
                425                 430                 435

atc agg tgc tcc gtg tagggatgct cgagactcaa ggccacatcc caagccagtg     1405
Ile Arg Cys Ser Val

            440

cgcactctga actagttttg catttgcgat ttcatgtatt taatgtgtgt gcgaacttat 1465

aattatttaa tgatgagacc tcgtatggaa taaaggacct ctgccgaatg tctgcttaca 1525

aaaaaaaaaa aaaa 1539

<210> 2
<211> 441
<212> PRT
<213> Drosophila melanogaster

<400> 2
Met Thr Thr Thr Pro Lys Ile Lys Ala Pro Val Ser Gly Pro Gly Leu
1               5                   10                  15

Pro Leu Leu Leu Gln Met Leu Met Gly Met Leu Leu Met Gly Leu Thr
            20                  25                  30

Ser Val Pro Gly Ala Thr Ala Thr Ala Asp Pro Lys Asn Ala Asn Val
        35                  40                  45

Lys Ala Leu Asp Arg Leu His Ala Gly Leu Phe Thr Asn Tyr Asp Ser
    50                  55                  60

Asp Val Gln Pro Val Phe Gln Gly Thr Pro Thr Asn Val Ser Leu Glu
65                  70                  75                  80

Met Val Val Thr Tyr Ile Asp Ile Asp Glu Leu Asn Gly Lys Leu Thr
                85                  90                  95

Thr His Cys Trp Leu Asn Leu Arg Trp Arg Asp Glu Glu Arg Val Trp
            100                 105                 110

Gln Pro Ser Gln Tyr Asp Asn Ile Thr Gln Ile Thr Leu Lys Ser Ser
        115                 120                 125

Glu Val Trp Thr Pro Gln Ile Thr Leu Phe Asn Gly Asp Glu Gly Gly
    130                 135                 140

Leu Met Ala Glu Thr Gln Val Thr Leu Ser His Asp Gly His Phe Arg
145                 150                 155                 160

Trp Met Pro Pro Ala Val Tyr Thr Ala Tyr Cys Glu Leu Asn Met Leu
                165                 170                 175

Asn Trp Pro His Asp Lys Gln Ser Cys Lys Leu Lys Ile Gly Ser Trp
            180                 185                 190

Gly Leu Lys Val Val Leu Pro Glu Asn Gly Thr Ala Arg Gly Glu Ser
        195                 200                 205

Leu Asp His Asp Asp Leu Val Gln Ser Pro Glu Trp Glu Ile Val Asp
    210                 215                 220

Ser Arg Ala His Phe Val Ser Gln Asp Tyr Tyr Gly Tyr Met Glu Tyr
225                 230                 235                 240

Thr Leu Thr Ala Gln Arg Arg Ser Ser Met Tyr Thr Ala Val Ile Tyr
                245                 250                 255

Thr Pro Ala Ser Cys Ile Val Ile Leu Ala Leu Ser Ala Phe Trp Leu
            260                 265                 270

Pro Pro His Met Gly Gly Glu Lys Ile Met Ile Asn Gly Leu Leu Ile
        275                 280                 285

Ile Val Ile Ala Ala Phe Leu Met Tyr Phe Ala Gln Leu Leu Pro Val
    290                 295                 300

Leu Ser Asn Asn Thr Pro Leu Val Val Ile Phe Tyr Ser Thr Ser Leu
305                 310                 315                 320

Leu Tyr Leu Ser Val Ser Thr Ile Val Glu Val Leu Val Leu Tyr Leu
                325                 330                 335

Ala Thr Gly Lys His Lys Arg Arg Leu Pro Glu Ala Leu Arg Lys Leu
            340                 345                 350

Leu His Gly His Leu Gly Thr Trp Leu Leu Leu Ser Val Phe Ser Thr
        355                 360                 365

Thr Gly Glu Ser Gln Ala Glu Lys Thr Lys Glu Met Asp Glu His Pro
    370                 375                 380

Tyr Glu Glu Ala Asp Glu Gln Glu Ser Ser Pro Leu Gly Ile Asn His
385                 390                 395                 400

Thr Glu Val Pro Gly Ala Lys Ala Asn Gln Phe Asp Trp Ala Leu Leu
                405                 410                 415

Ala Thr Ala Val Asp Arg Ile Ser Phe Val Ser Phe Ser Leu Ala Phe
            420                 425                 430

Leu Ile Leu Ala Ile Arg Cys Ser Val
        435                 440

<210> 3
<211> 20
<212> DNA
<213> Künstliche Sequenz

<220>
<223> Beschreibung der künstlichen Sequenz:primer

<400> 3
tggcarccnt cncartayga                                                    20

<210> 4
<211> 21
<212> DNA
<213> Künstliche Sequenz

<220>
<223> Beschreibung der künstlichen Sequenz:primer

<400> 4
catratytty tcnccnccca t                                                  21

## Beschreibung

Die Erfindung betrifft Nukleinsäuren, die für Acetylcholinrezeptor-β-Untereinheiten von Insekten kodieren, sowie Polypeptide, welche die biologische Funktion solcher Acetylcholinrezeptor-β-Untereinheiten ausüben, und insbesondere deren Verwendung zum Auffinden von Wirkstoffen für den Pflanzenschutz.

Nikotinische Acetylcholinrezeptoren sind ligandengesteuerte Ionenkanäle, die eine Rolle bei der Neurotransmission im Tierreich spielen. Die Bindung von Acetylcholin oder anderen Agonisten an den Rezeptor verursacht eine vorübergehende Öffnung des Kanals und gestattet den Durchstrom von Kationen. Man nimmt an, dass ein Rezeptor aus fünf Untereinheiten besteht, die sich um eine Pore gruppieren. Jede dieser Untereinheiten ist ein Protein, das aus einem extrazellulären N-terminalen Teil besteht, gefolgt von drei Transmembranregionen, einem intrazellulären Teil, sowie einer vierten Transmembranregion und einem kurzen extrazellulären C-terminalen Teil. Bestimmte Untereinheiten tragen auf ihrem extrazellulären Teil die Bindestelle für Liganden wie Acetylcholin. Zwei vicinale Cysteine sind Bestandteil dieser Bindestelle und daher gemeinsames Strukturmerkmal aller ligandenbindenden Untereinheiten, die auch als α-Untereinheiten bezeichnet werden. Untereinheiten ohne dieses Strukturmerkmal werden je nach Lokalisation und Funktion des Rezeptors als β-, γ-, δ- oder ε-Untereinheiten bezeichnet (Changeux et al. 1992).

Acetylcholinrezeptoren sind vor allem in Wirbeltieren gut untersucht. Anhand ihrer anatomischen Lokalisierung und ihrer funktionellen Eigenschaften (Leitungseigenschaften des Kanals, Desensibilisierung, Sensitivität gegenüber Agonisten und Antagonisten, sowie gegenüber Toxinen wie z.B. α-Bungarotoxin) lassen sich hier drei Gruppen unterscheiden. Die Einteilung korreliert mit der molekularen Zusammensetzung der Rezeptoren. Es gibt heterooligomere Rezeptoren mit der Untereinheitenzusammensetzung α₂βγδ, die im Muskel vorkommen (Noda et al. 1982, Claudio et al. 1983, Devillers-Thiery et al. 1983, Noda et al. 1983a, b), heterooligomere Rezeptoren, die Untereinheiten aus der Gruppe α2 - α6 und β2 - β4 enthalten, und die im Nervensystem vorkommen ( Schoepfer et al. 1990, Heinemann et al. 1997), sowie homooligomere Rezeptoren, die Untereinheiten aus der Gruppe α7 - α9 enthalten, und die ebenfalls im Nervensystem vorkommen (Lindstrom et al. 1997, Elgoyhen et al. 1997). Diese Einteilung wird auch durch eine Betrachtung der Verwandschaft der Gensequenzen der verschiedenen Untereinheiten gestützt. Typischerweise sind die Sequenzen funktionell homologer Untereinheiten verschiedener Spezies ähnlicher als Sequenzen von Untereinheiten aus verschiedenen Gruppen, aber der gleichen Spezies. Weiterhin sind die Gensequenzen aller bekannten Acetylcholinrezeptor-Untereinheitenen nicht nur untereinander in gewissem Maße ähnlich, sondern auch mit denen einiger anderer ligandengesteuerter Ionenkanäle (z.B. den Serotoninrezeptoren vom Typ 5HT₃, den GABA-gesteuerten Chloridkanälen, den Glycin-gesteuerten Chloridkanälen). Man geht daher davon aus, dass alle diese Rezeptoren von einem gemeinsamen Vorläufer abstammen und ordnet sie in eine Supergenfamilie ein (Ortells et al. 1995).

In Insekten ist Acetylcholin der wichtigste exzitatorische Neurotransmitter des zentralen Nervensystems. Dementsprechend lassen sich Acetylcholinrezeptoren an Präparaten zentraler Ganglien aus Insekten elektrophysiologisch nachweisen. Der Nachweis gelingt sowohl an post- als auch an präsynaptischen Nervenendigungen sowie an den Zellkörpern von Interneuronen, Motorneuronen und modulatorischen Neuronen (Breer et al. 1987, Buckingham et al. 1997). Unter den Rezeptoren gibt es solche, die durch α-Bungarotoxin inhibiert werden, und solche, die insensitiv sind (Schloß et al. 1988). Die Acetylcholinrezeptoren sind außerdem der molekulare Angriffspunkt wichtiger natürlicher (z.B. Nikotin) und synthetischer Insektizide (z.B. Chloronikotinyle).

Die Gensequenzen einer Anzahl von nikotinischen Acetylcholinrezeptoren der Insekten ist bereits bekannt. So sind für Drosophila melanogaster die Sequenzen fünf verschiedener Untereinheiten beschrieben (Bossy et al. 1988, Hermanns-Borgmeyer et al. 1986, Sawruk et al. 1990a, 1990b, Schulz et al. 1998); für Locusta migratoria sind ebenfalls fünf (Hermsen et al. 1998), für Schistocerca gregaria eine (Marshall et al. 1990), für Myzus persicae sechs (Sgard et al. 1998, Huang et al. 1999), für Manduca sexta zwei (Eastham et al. 1997, Genbank AJ007397) und für Heliothis virescens sechs (DE 198 19 829, Genbank AF143846, AF143847, AJ000399, AF096878, AF096879) Sequenzen beschrieben. Zudem ist eine Reihe von partiellen Gensequenzen aus Drosophila melanogaster als sog. expressed sequence tags charakterisiert worden (Genbank AA540687, AA698155, AA697710, AA697326). Diese Sequenzen können in α- und β-Untereinheiten klassifiziert werden, je nachdem, ob die zwei vicinalen Cysteine der Ligandenbindestelle vorhanden sind oder nicht. Während aber in Vertebraten insgesamt vier verschiedene β-Untereinheiten bekannt sind (Schoepfer et al. 1990, Heinemann et al. 1997), in Caenorhabditis elegans mindestens fünf (Bargmann und Kaplan 1998), ist in jeder der untersuchten Insektenspezies mit Ausnahme von Drosophila melanogaster jeweils nur eine β-Untereinheit identifiziert worden (Huang et al., 1999, Hermsen et al. 1998, Genbank AJ007397). Bei dieser weit verbreiteten Untereinheit handelt es sich um ein Homologes der Untereinheit, die bei Drosophila melanogaster als ARD bezeichnet wird (Schloß et al. 1988). Die andere β-Untereinheit von Drosophila, SBD (Sawruk et el. 1990b), ist in ihrer Sequenz den α-Untereinheiten ähnlicher als der anderen β-Untereinheit, besitzt aber nicht die vicinalen Cysteine.

Die rekombinante Expression nikotinischer Rezeptoren aus Insekten hat sich als schwieriger erwiesen als die der analogen Rezeptoren aus Vertebraten oder C. elegans. So ist es bisher im allgemeinen nicht gelungen, nikotinische Rezeptoren, die nur aus Untereinheiten von Insekten bestehen, so zu exprimieren, dass ihre funktionalen Eigenschaften wie z.B. Empfindlichkeit denen natürlicher Rezeptoren gleichen (Amar et al. 1995, Hermsen et al. 1998, Sgard et al. 1998). Zumindest einige α-Untereinheiten aus verschiedenen Insektenspezies tragen jedoch zu einem funktionellen Rezeptor bei, wenn statt einer Insekten-β-Untereinheit eine β2-Untereinheit des Huhns in Xenopus-Oocyten koexprimiert wird (Marshall et al. 1990, Schulz et al. 1998, Matsuda et al. 1998). Dies, und die Tatsache, dass im wesentlichen nur eine β-Untereinheit aus Insekten bekannt ist, lässt die Vermutung zu, dass weitere, bisher unbekannte β-Untereinheiten vorliegen.

Es ist von großer praktischer Bedeutung, beispielsweise für die Suche nach neuen Insektiziden, nikotinische Rezeptoren aus Insekten in eukaryotischen Zelllinien oder in Oocyten von Xenopus laevis funktionell auszuprägen.

Der vorliegenden Erfindung liegt somit insbesondere die Aufgabe zu Grunde, Nukleinsäuren zur Verfügung zu stellen, die für neue Acetylcholinrezeptor-β-Untereinheiten von Insekten kodieren.Diese neuen Nukleinsäuren sollen die rekombinante Ausprägung von nikotinischen Acetylcholinrezeptoren ermöglichen, die ausschließlich aus Untereinheiten von Insekten bestehen.

Die Aufgabe wird gelöst durch die Bereitstellung einer Nukleinsäure umfassend eine Sequenz ausgewählt aus
(a) der Sequenz gemäß SEQ ID NO: 1,
(b) zumindest 14 Basenpaare langen Teilsequenzen der unter (a) definierten Sequenz,
(c) Sequenzen, welche an die unter (a) definierte Sequenz hybridisieren,
(d) Sequenzen, welche eine zumindest 70 %ige Identität mit der Sequenz zwischen Position 43 und Position 1368 der unter (a) definierten Sequenz aufweisen,
(e) Sequenzen, welche zu der unter (a) definierten Sequenz komplementär sind und
(f) Sequenzen, welche aufgrund der Degeneriertheit des genetischen Codes für dieselbe Aminosäuresequenz kodieren wie die unter (a) bis (d) definierten Sequenzen.

Bei den erfindungsgemäßen Nukleinsäuren handelt es sich insbesondere um einzelsträngige oder doppelsträngige Desoxyribonukleinsäuren (DNA) oder Ribonukleinsäuren (RNA). Bevorzugte Ausführungsformen sind Fragmente genomischer DNA, die Introns enthalten können, und cDNAs.

Eine bevorzugte Ausführungsform der erfindungsgemäßen Nukleinsäuren stellt die cDNA dar, welche die Nukleinsäuresequenz gemäß SEQ ID NO: 1 besitzt.

Der Grad der Identität der Nukleinsäuresequenzen wird vorzugsweise bestimmt mit Hilfe des Programms GAP aus dem Programmpaket GCG, Version 10.0 unter Standardeinstellungen (Devereux et al. 1984).

Der Ausdruck "hybridisieren", wie er hierin verwendet wird, beschreibt den Vorgang, bei welchem ein einzelsträngiges Nukleinsäuremolekül mit einem komplementären Strang eine Basenpaarung eingeht. Auf diese Weise können ausgehend von der hierin offenbarten Sequenzinformation beispielsweise DNA-Fragmente aus anderen Insekten als Drosophila melanogaster isoliert werden, welche für Polypeptide mit der biologischen Funktion von Acetylcholinrezeptor-β-Untereinheiten kodieren.

Bevorzugte Hybridisierungsbedingungen sind nachstehend angegeben:
Hybridisierungslösung: 6X SSC /0 % Formamid, bevorzugte Hybridisierungslösung: 6X SSC / 25 % Formamid
Hybridisierungstemperatur: 34°C, bevorzugte Hybridisierungstemperatur: 42°C

1. Waschschritt: 2X SSC bei 40°C,
2. Waschschritt: 2X SSC bei 45°C; bevorzugter 2. Waschschritt: 0,6X SSC bei 55°C; besonders bevorzugter 2. Waschschritt: 0,3X SSC bei 65°C.

Von der vorliegenden Erfindung sind Nukleinsäuren umfasst, die eine zumindest 70 %ige Identität, vorzugsweise eine zumindest 80 %ige Identität, besonders bevorzugt eine zumindest 90 %ige Identität, ganz besonders bevorzugt eine zumindest 95 %ige Identität, mit der Sequenz zwischen Position 43 und Position 1368 der Sequenz gemäß SEQ ID NO: 1 vorzugsweise über eine Länge von wenigstens 100, besonders bevorzugt wenigstens 500 fortlaufenden Nukleotiden und ganz besonders bevorzugt über die Gesamtlänge aufweisen.

Gegenstand der Erfindung sind weiterhin Vektoren, die zumindest eine der erfindungsgemäßen Nukleinsäuren enthalten. Als Vektoren können alle in molekularbiologischen Laboratorien verwendete Plasmide, Phasmide, Cosmide, YACs oder künstliche Chromosomen verwendet werden. Für die Expression der erfindungsgemäßen Nukleinsäure kann diese mit üblichen regulatorischen Sequenzen verknüpft werden.
Die Auswahl solcher regulatorischen Sequenzen ist davon abhängig, ob zur Expression pro- oder eukaryotische Zellen bzw. zellfreie Systeme verwendet werden. Besonders bevorzugt als Expressionskontrollsequenz sind z.B. der frühe oder späte Promotor des SV40 oder des Adenovirus, des Cytomegalovirus, das lac-System, das trp-System, die Haupt-Operator- und Promotorregionen des Phagen lambda, die Kontrollregionen des fd-Hüllproteins, der Promotor der 3-Phosphoglyceratkinase, der Promotor der Sauren Phosphatase und der Promotor des α-Mating-Faktors der Hefe, der immediate early Promoter aus Baculovirus, der Metallothionin Promoter aus Drosophila melanogaster.

Zur Expression der erfindungsgemäßen Nukleinsäure kann diese in geeignete Wirtszellen eingebracht werden. Als Wirtszellen eignen sich sowohl prokaryotische Zellen, vorzugsweise E.coli, als auch eukaryotische Zellen, vorzugsweise Säuger- oder Insektenzellen. Weitere Beispiele für geeignete einzellige Wirtszellen sind: Pseudomonas, Bacillus, Streptomyces, Hefen, HEK-293, Schneider S2, SF9, CHO-, COS1-, COS7-, Zellen, Pflanzenzellen in Zellkultur sowie Amphibienzellen, insbesondere Oocyten.

Gegenstand der vorliegenden Erfindung sind auch die Polypeptide, die von der erfindungsgemäßen Nukleinsäure kodiert werden.

Weiterhin sind Polypeptide Gegenstand der vorliegenden Erfindung, welche eine Aminosäuresequenz umfassen, die eine zumindest 40 %ige Identität, vorzugsweise eine zumindest 60 %ige Identität, besonders bevorzugt eine zumindest 80 %ige Identität, mit der Sequenz gemäß SEQ ID NO: 2 über eine Länge von wenigstens 20, vorzugsweise wenigstens 25, besonders bevorzugt wenigstens 30 fortlaufenden Aminosäuren und ganz besonders bevorzugt über die Gesamtlänge aufweisen.

Der Grad der Identität der Aminosäuresequenzen wird vorzugsweise bestimmt mit Hilfe des Programms GAP aus dem Programmpaket GCG, Version 10.0 unter Standardeinstellungen (Devereux et al. 1984).

Gegenstand der vorliegenden Erfindung sind weiterhin Acetylcholinrezeptoren, die die erfindungsgemäßen Polypeptide umfassen.

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch chemische Verfahren, die Stand der Technik sind, modifiziert sein können. Solche Modifikationen können an verschiedenen Stellen und mehrfach in einem Polypeptid vorkommen, wie beispielsweise an dem Peptid-Rückgrat, an der Aminosäure-Seitenkette, am Amino-und/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phosphatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristylierungen, Oxidationen, proteolytische Prozessierungen, Phosphorylierungen, Selenylierungen und tRNA-vermittelte Additionen von Aminosäuren.

Die erfindungsgemäßen Polypeptide können in der Form "reifer" Proteine oder als Teile größerer Proteine, z.B. als Fusionsproteine, vorliegen. Weiterhin können sie Sezernierungs- oder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen.

Die erfindungsgemäßen Polypeptide müssen nicht vollständige Acetylcholinrezeptor-β-Untereinheiten darstellen, sondern können auch nur Fragmente davon sein, solange sie zumindest noch die biologische Funktion der vollständigen Untereinheiten ausüben können.

Die erfindungsgemäßen Polypeptide müssen nicht von Acetylcholinrezeptor-β-Untereinheiten aus Drosophila melanogaster ableitbar sein. Als erfindungsgemäß werden auch Polypeptide betrachtet, die Acetylcholinrezeptor-β-Untereinheiten von anderen Insekten entsprechen oder Fragmente davon, die noch die biologische Funktion dieser Untereinheiten ausüben können.

Die erfindungsgemäßen Polypeptide können im Vergleich zu der entsprechenden Region natürlich vorkommender Acetylcholinrezeptor-β-Untereinheiten Deletionen oder Aminosäuresubstitutionen aufweisen, solange sie zumindest noch die biologische Funktion der vollständigen Untereinheiten ausüben. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Die folgende Liste zeigt bevorzugte konservative Substitutionen:

| **Ursprünglicher Rest** | **Substitution** |
|---|---|
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |
| Trp | Tyr |
| Tyr | Trp, Phe |
| Val | Ile, Leu |

Der Ausdruck "biologische Funktion einer Acetylcholinrezeptor-β-Untereinheit", wie er hierin verwendet wird, bedeutet eine Beteiligung am Aufbau von funktionsfähigen Acetylcholinrezeptoren, d.h. die Fähigkeit, mit anderen Untereinheiten der Rezeptoren interagieren zu können.

Eine bevorzugte Ausführungsform der erfindungsgemäßen Polypeptide stellt eine Acetylcholinrezeptor-β-Untereinheit von Drosophila melanogaster dar, welche die Aminosäuresequenz gemäß SEQ ID NO: 2 besitzt.

Gegenstand der vorliegenden Erfindung sind weiterhin Verfahren zum Herstellen der erfindungsgemäßen Polypeptide. Zur Herstellung der Polypeptide, die von der erfindungsgemäßen Nukleinsäure kodiert werden, können Wirtszellen, die die erfindungsgemäße Nukleinsäure enthalten, unter geeigneten Bedingungen kultiviert werden. Die gewünschten Polypeptide können danach auf übliche Weise aus den Zellen oder dem Kulturmedium isoliert werden.

Ein schnelles Verfahren zum Isolieren der erfindungsgemäßen Polypeptide, die von Wirtszellen unter Verwendung einer erfindungsgemäßen Nukleinsäure synthetisiert werden, beginnt mit der Expression eines Fusionsproteins, wobei der Fusionspartner auf einfache Weise affinitätsgereinigt werden kann. Der Fusionspartner kann beispielsweise Glutathion S-Transferase sein. Das Fusionsprotein kann dann an einer Glutathion-Affinitätssäule gereinigt werden. Der Fusionspartner kann durch partielle proteolytische Spaltung beispielsweise an Linkern zwischen dem Fusionspartner und dem zu reinigenden erfindungsgemäßen Polypeptid abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren auf präparativer Elektrophorese, FPLC, HPLC (z.B. unter Anwendung von Gelfiltrations-, Reversphasen- oder leicht hydrophoben Säulen), Gelfiltration, differentieller Präzipitation, Ionenaustausch-Chromatographie und Affinitätschromatographie.

Die Reinigung der erfmdungsgemäßen Polypeptide kann die Isolierung von Membranen ausgehend von Wirtszellen, die die erfindungsgemäßen Nukleinsäuren exprimieren, umfassen. Vorzugsweise exprimieren solche Zellen die erfindungsgemäßen Polypeptide in einer ausreichenden Kopienanzahl, so dass die Menge der Polypeptide in einer Membranfraktion mindestens 10-fach höher ist als diejenige, die in vergleichbaren Membranen von Zellen gefunden wird, die Acetylcholinrezeptoren natürlicherweise exprimieren; besonders bevorzugt ist Menge mindestens 100-fach, ganz besonders bevorzugt mindestens 1000-fach höher.

Die Ausdrücke "Isolierung oder Reinigung", wie sie hierin verwendet werden, bedeuten, dass die erfindungsgemäßen Polypeptide von anderen Proteinen oder anderen Makromolekülen der Zelle oder des Gewebes abgetrennt werden. Vorzugsweise ist eine die erfindungsgemäßen Polypeptide enthaltende Zusammensetzung hinsichtlich des Proteingehalts gegenüber einer Präparation aus den Wirtszellen mindestens 10-fach und besonders bevorzugt mindestens 100-fach angereichert.

Die erfindungsgemäßen Polypeptide können auch ohne Fusionspartner mit Hilfe von Antikörpern, die an die Polypeptide binden, affinitätsgereinigt werden.

Weiterhin sind Antikörper Gegenstand der Erfindung, die spezifisch an die vorstehend genannten Polypeptide bzw. Rezeptoren binden. Die Herstellung solcher Antikörper erfolgt auf die übliche Weise. Beispielsweise können solche Antikörper produziert werden durch die Injektion eines substantiell immunkompetenten Wirts mit einer für die Antikörperproduktion effektiven Menge eines erfindungsgemäßen Acetylcholinrezeptor-Polypeptids oder eines Fragments davon und durch nachfolgende Gewinnung dieses Antikörpers. Weiterhin lässt sich in an sich bekannter Weise eine immortalisierte Zellinie erhalten, die monoklonale Antikörper produziert. Die Antikörper können gegebenenfalls mit einem Nachweisreagenz markiert sein.

Bevorzugte Beispiele für ein solches Nachweis-Reagenz sind Enzyme, radioaktiv markierte Elemente, fluoreszierende Chemikalien oder Biotin. Anstelle des vollständigen Antikörpers können auch Fragmente eingesetzt werden, die die gewünschten spezifischen Bindungseigenschaften besitzen.

Die erfindungsgemäße Nukleinsäure kann insbesondere zur Herstellung transgener Invertebraten verwendet werden. Diese können in Testsysteme eingesetzt werden, die auf einer vom Wildtyp abweichenden Expression der erfindungsgemäßen Rezeptoren oder Varianten hiervon basieren. Ferner fallen hierunter sämtliche transgenen Invertebraten, bei denen durch die Modifikation anderer Gene oder Genkontrollsequenzen (Promotoren) eine Veränderung der Expression der erfindungsgemäßen Rezeptoren oder deren Varianten eintritt.

Die Herstellung der transgenen Invertebraten erfolgt beispielsweise in Drosophila melanogaster durch P-Element-vermittelten Gentransfer (Hay et al. 1997) oder in Caenorhabditis elegans durch Transposon-vermittelten Gentransfer (z.B. durch Tcl, Plasterk 1996).

Gegenstand der Erfindung sind somit auch transgene Invertebraten, die zumindest eine der erfindungsgemäßen Nukleinsäuren enthalten, vorzugsweise transgene Invertebraten der Arten Drosophila melanogaster oder Caenorhabditis elegans, sowie deren transgene Nachkommen. Vorzugsweise enthalten die transgenen Invertebraten die erfindungsgemäßen Rezeptoren in einer vom Wildtyp abweichenden Form.

Die erfindungsgemäße Nukleinsäure kann auf die übliche Weise hergestellt werden.
Beispielsweise kann das Nukleinsäuremolekül vollständig chemisch synthetisiert werden. Man kann auch nur kurze Stücke der erfindungsgemäßen Sequenz chemisch synthetisieren und solche Oligonukleotide radioaktiv oder mit einem Fluoreszenzfarbstoff markieren. Die markierten Oligonukleotide können verwendet werden, um ausgehend von Insekten-mRNA hergestellte cDNA-Banken zu durchsuchen. Klone, an die die markierten Oligonukleotide hybridisieren, werden zur Isolierung der betreffenden DNA ausgewählt. Nach der Charakterisierung der isolierten DNA erhält man auf einfache Weise die erfindungsgemäße Nukleinsäure.

Die erfindungsgemäße Nukleinsäure kann auch mittels PCR-Verfahren unter Verwendung chemisch synthetisierter Oligonukleotide hergestellt werden.

Der Ausdruck "Oligonukleotid(e)", wie er hierin verwendet wird, bedeutet DNA-Moleküle, die aus 10 bis 50 Nukleotiden, vorzugsweise 15 bis 30 Nukleotiden, bestehen. Sie werden chemisch synthetisiert und können als Sonden verwendet werden.

Die erfindungsgemäße Nukleinsäure kann zur Isolierung und Charakterisierung der regulatorischen Regionen, die natürlicherweise benachbart zu der kodierenden Region vorkommen, verwendet werden. Solche regulatorischen Regionen sind somit ebenfalls Gegenstand der vorliegenden Erfindung.

Mit Hilfe der erfindungsgemäße Nukleinsäure können neue Wirkstoffe für den Pflanzenschutz bzw. pharmazeutische Wirkstoffe für die Behandlung von Mensch und/oder Tier, wie Verbindungen, welche als Modulatoren, insbesondere als Agonisten oder Antagonisten, die Leitungseigenschaften der erfindungsgemäßen Acetylcholinrezeptoren verändern, identifiziert werden. Dazu wird ein rekombinantes DNA-Molekül, das die erfindungsgemäße Nukleinsäure umfasst, in eine geeignete Wirtszelle eingebracht. Die Wirtzelle wird in Gegenwart einer Verbindung oder einer Probe, welche eine Vielzahl von Verbindungen umfasst, unter Bedingungen kultiviert, die die Expression der erfindungsgemäßen Rezeptoren erlauben. Eine Veränderung der Rezeptoreigenschaften kann - wie nachstehend in Beispiel 2 beschrieben - detektiert werden. Auf diese Weise ist es möglich, insektizide Substanzen aufzufinden.

Die erfindungsgemäße Nukleinsäure ermöglicht auch das Auffinden von Verbindungen, die an die erfindungsgemäßen Rezeptoren binden. Diese können ebenfalls als Insektizide angewandt werden. Beispielsweise werden Wirtszellen, die die erfindungsgemäße Nukleinsäure enthalten und die entsprechenden Rezeptoren bzw. Polypeptide exprimieren oder die Genprodukte selbst mit einer Verbindung oder einem Gemisch von Verbindungen unter Bedingungen in Kontakt gebracht, die die Wechselwirkung zumindest einer Verbindung mit den Wirtszellen, den Rezeptoren oder den einzelnen Polypeptiden erlauben.

Unter Verwendung von Wirtszellen oder transgenen Invertebraten, die die erfindungsgemäße Nukleinsäure enthalten, ist es auch möglich, Substanzen aufzufinden, welche die Expression der Rezeptoren verändern.

Die vorstehend beschriebenen erfindungsgemäße Nukleinsäure, Vektoren und regulatorischen Regionen können außerdem zum Aufffinden von Genen verwendet werden, die für Polypeptide kodieren, welche am Aufbau von funktionell ähnlichen Acetylcholinrezeptoren in Insekten beteiligt sind. Unter funktionell ähnlichen Rezeptoren werden gemäß der vorliegenden Erfindung Rezeptoren verstanden, die Polypeptide umfassen, welche sich zwar hinsichtlich der Aminosäuresequenz von den hierin beschriebenen Polypeptiden unterscheiden, aber im wesentlichen dieselben Funktionen haben.

### Erläuterungen zum Sequenzprotokoll und zu der Abbildung:

SEQ ID NO: 1 zeigt die Nukleotidsequenz der isolierten Db3-cDNA, beginnend mit Position 1 und endend mit Position 1539. SEQ ID NO: 1 und SEQ ID NO: 2 zeigen ferner die Aminosäuresequenzen des von der Db3-cDNA-Sequenz abgeleiteten Proteins.

SEQ ID NO: 3 und SEQ ID NO: 4 zeigen die im Beispiel 1 beschriebenen Oligodesoxynukleotide.

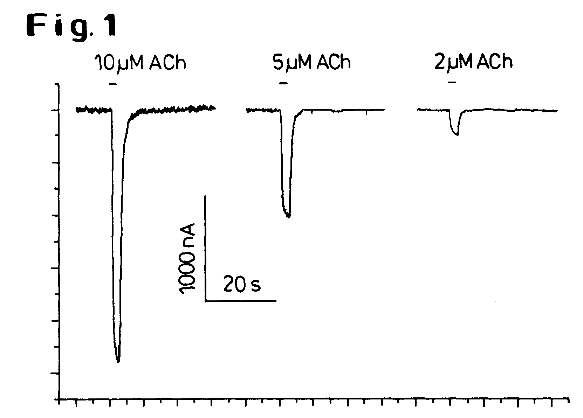

Abb. 1 zeigt die Acetylcholin-induzierten Ströme, die in Xenopus-Oocyten mit Hilfe von Ganz-Zell-Ableitungen gemessen wurden, aufgetragen gegen die Zeit. Ströme werden in nano-Ampere, Zeit in Sekunden dargestellt. Die Oocyten waren mit cDNA-Expressionsplasmiden injiziert, die für die α1-, α2- and β3-Untereinheit von Drosophila kodierten. Die Zeitpunkte der Acetylcholin-Applikationen sind mit Querbalken gekennzeichnet.

### Beispiele:

### Beispiel 1

### Isolierung der beschriebenen Polynukleotidsequenz

### Allgemeines

Die Manipulation von Polynukleotiden erfolgte nach Standardmethoden der rekombinanten DNA Technologie (Sambrook, et al., 1989). Die bioinformatische Bearbeitung von Nukleotid- und Proteinsequenzen erfolgten mit dem Programmpaket GCG Version 10.0 (GCG Genetics Computer Group, Inc., Madison Wisconsin, USA).

### Isolierung partieller Polynukleotidsequenzen mittels PCR

Aus einer Datenbankrecherche mit der Proteinsequenz der ARD Untereinheit von Drosophila melanogaster gegen die genomische Drosophila melanogaster Datenbank wurden ein Nukleinsäurebereich identifiziert, der eine Identität auf Aminosäureebene zu ARD von 28% besitzt. Anhand dieser Partialsequenz wurden Oligodesoxynukleotidprimer (dglsense: 5'-TGGCARCCITCICARTAYGA-3', dg2anti: 5'-CATRATYTTYTCICCICCCAT-3') abgeleitet. RNA wurde mittels Trizol-Reagenz (Gibco BRL) aus Drosophila melanogaster-Embryonen nach Angaben des Herstellers isoliert. 10 µg dieser RNA wurden in eine cDNA-Erststrangsynthese (Superscript Präamplifizierungssystem für die cDNA-Erststrangsynthese, Gibco BRL, nach Angaben des Herstellers, 45°C Reaktionstemperatur) eingesetzt. Anschließend wurden jeweils 1/100 der o.g. Erststrang-cDNA in eine Polymerasekettenreaktion (PCR) mit den Oligonukleotiden dglsense und dg2anti eingesetzt (Taq DNA Polymerase, rekombinant, Gibco BRL). Die PCR-Parameter waren wie folgt: 94°C, 1 min; 35 mal (94°C, 30 s; 55°C, 30 s; 72°C, 45 s) Hieraus ergab sich eine im Agarosegel (1 %) erkennbare Bande von ca. 0,6 kb. Diese wurde mittels des pCR TOPO Kits subkloniert (Invitrogen).

Isolierung von poly-A-enthaltender RNA aus Drosophila melanogaster-Gewebe und Konstruktion der cDNA-Bibliotheken

Die RNA für die cDNA-Bibliothek wurde aus Drosophila melanogaster Embryonen und Larven mit Trizol (Gibco BRL) nach Angaben des Herstellers isoliert. Aus dieser RNA wurden nun die poly-A-enthaltenden RNAs durch Reinigung über Dyna Beads 280 (Dynal) isoliert. 5 µg dieser poly A enthaltenden RNAs wurden anschließend in die Konstruktion der cDNA-Bibliothek mit dem λ-ZAP-CMV Vektor eingesetzt (cDNA Synthesis Kit, ZAP-cDNA Synthesis Kit und ZAP-cDNA Gigapack III Gold Cloning Kit, alle Stratagene).

### Isolierung der vollständige Polynukleotidsequenzen aus der cDNA-Bibliothek

Der Screen mit 10⁶ plaque forming units wurde mit Hilfe des DIG Systems durchgeführt (alle Reagenzien und Verbrauchsmaterialien Boehringer Mannheim, nach Angaben im "The DIG System User's Guide for Filter Hybridization", Boehringer Mannheim). Die eingesetzte DNA-Sonde wurden durch PCR mittels Digoxygenin markiertem dUTP präpariert, Die Hybridisierungen erfolgten in DIG Easy Hyb (Boehringer Mannheim) bei 42°C über Nacht. Der Nachweis markierter DNA auf Nylonmembranen geschah durch Chemolumineszenz (CDP-Star, Boehringer Mannheim) unter Verwendung von Röntgenfilmen (Hyperfilm MP, Amersham). Vereinzelte und in der Hybridisierung positive Plaques wurden mittels in vivo Exzision in Plasmide (pCMV) überführt (Stratagene, ZAP-cDNA Synthesis Kit). Die isolierten Plasmide wurden zur Identifikation mittels T3 und T7 Primer ansequenziert (ABI Prism Dye Terminator Cycle Sequencing Kit, ABI, mit ABI Prism 310 Genetic Analyzer). Die Bestimmung der vollständigen Polynukleotidsequenzen des DB3 erfolgte durch Primer Walking mittels Cycle Sequencing ABI Prism Dye Terminator Cycle Sequencing Kit, ABI, mit ABI Prism 310 Genetic Analyzer.

### Beispiel 2

Expression von rekombinanten Insekten-Acetylcholinrezeptoren enthaltend die neue β3-Untereinheit von Drosophila in Xenopus-Oocyten

Oocyten wurden gleichzeitig mit cDNA-Expressionsplasmiden injiziert, die für die α1-, α2- and β3-Untereinheit von Drosophila kodierten. Die α-Untereinheiten waren in pcDNA3 kloniert, die β-Untereinheit in pCMV wie oben beschrieben. Nach drei bis fünf Tagen Inkubation wurden die Ströme durch die Zellmembran der Oocyten mit Ganz-Zell-Ableitungen gemessen wie beschrieben (Cooper et al. 1996). Dazu wurde die Potentialdifferenz über die Zellmembran bei -80mV konstant gehalten und die Zellen mit Acetylcholin (10µM) stimuliert. Unmittelbar nach dem Stimulus konnten starke Einwärtsströme gemessen werden, die typisch für die Aktivierung von Ionenkanälen waren (Abb. 1). Dies zeigt, dass die neue β3-Untereinheit von Drosophila funktionale Rezeptoren mit einer der beiden koinjizierten α-Untereinheiten bildet, oder mit beiden.

### Literatur:

Amar et al. (1995), A nicotinic acetylcholine receptor subunit from insect brain forms a non-desensitizing homo-oligomeric nicotinic acetylcholine receptor when expressed in Xenopus oocytes, Neuroscience Letters 199, 107-110

Bargmann und Kaplan (1998), Signal transduction in the Caenorhabditis elegans nervous system, Ann. Rev. Neurosci., 21, 279-308

Bossy et al. (1988), Conservation of neural nicotinic acetylcholine receptors from Drosophila to vertebrate central nervous systems, EMBO J. 7, 611-618

Breer et al. (1987), Molecular properties and functions of insect acetylcholine receptors, J. Insect Physiol. 33, 771-790

Buckingham et al. (1997), Imidacloprid actions on insect neuronal acetylcholine receptors, J. Exp. Biol. 200, 2685-2692

Changeux et al. (1992), The functional architecture of the nicotinic acetylcholine receptor explored by affinity labelling and site-directed mutagenesis, Quarterly Review of Biophysics 25, 395-432

Claudio et al. (1983), Nucleotide and deduced amino acid sequences of Torpedo californica acetylcholine receptor γ subunit, Proc. Natl. Acad. Sci. USA 80, 1111-1115

Cooper et al. (1996), Eur. J. Pharmacol. 309, 287

Devereux et al. (1984), Nucleic Acids Research 12, 387

Devillers-Thiery et al. (1983), Complete mRNA coding sequence of the acetylcholine binding α-subunit of Torpedo marmorata acetylcholine receptor: a model for the transmembrane organization of the polypeptide chain, Proc. Natl. Acad. Sci. USA 80, 2067-2071

Elgoyhen et al. (1997), US Pat. No. 5,683,912

Eastham et al. (1998), Characterisation ofa nicotinic acetylcholine receptor from the insect Manduca sexta, Eur. J. Neurosci 10, 879-889

Hay et al. (1997), P element insertion-dependent gene activation in the Drosophila eye, Proceedings of The National Academy of Sciences of The United States of America 94 (10), 5195-5200

Hermans-Borgmeyer et al. (1986), Primary structure of a developmentally regulated nicotinic acetylcholine receptor protein from Drosophila, EMBO J. 5, 1503-1508

Hermsen et al. (1998), Neuronal nicotinic receptors in the locust Locusta migratoria. Cloning and expression, J. Biol. Chem. 17, 18394-404.

Heinemann et al. (1997), US Pat. No. 5,591,590

Huang et al. (1999), Molecular characterization and imidacloprid selectivity of nicotinic acetylcholine receptor subunits from the peach-potato aphid Myzus persicae, Neurochem., 73, 380-389

Lindstrom et al. (1997), US Pat. No. 5,599,709

Marshall et al. (1990), Sequence and functional expression of a single α subunit of an insect nicotinic acetylcholine receptor, EMBO J. 9, 4391-4398

Matsuda et al. (1998), Effects of the a subunit on imidacloprid sensitivity of recombinant nicotinic acetylcholine receptors, Br. J. Pharmacol. 123, 518-524

Noda et al. (1982), Primary structure of a-subunit precursor of Torpedo californica acetylcholine receptor deduced from cDNA sequence, Nature 299, 793-797

Noda et al. (1983a), Primary structures of β- and δ-subunit precursor of Torpedo californica acetylcholine receptor deduced from cDNA sequences, Nature 301, 251- 255

Noda et al. (1983b), Structural homology of Torpedo californica acetylcholine receptor subunits, Nature 302, 528-532

Ortells et al. (1995), Evolutionary history of the ligand-gated ion-channel superfamily of receptors, Trends in Neurosience 18, 121-127

Plasterk (1996), The Tcl/mariner transposon family, Transposable Elements/Current Topics in Microbiology and Immunology 204, 125-143

Sambrook et al. (1989), Molecular Cloning, A Laboratory Manual, 2nd ed. Cold Spring Harbour Press

Sawruk et al. (1990a), Heterogeneity of Drosophila nicotinic acetylcholine receptors: SAD, a novel developmentally regulated α-subunit, EMBO J. 9, 2671-2677

Sawruk et al. (1990b), SBD, a novel structural subunit of the Drosophila nicotinic acetylcholine receptor, shares its genomic localization with two α-subunits, FEBS Lett. 273, 177-181

Schloß et al. (1988), Neuronal acetylcholine receptors of Drosophila: the ARD protein is a component of a high-affinity α-bungarotoxin binding complex, EMBO J 7, 2889-2984

Schoepfer et al. (1990), Brain alpha-bungarotoxin binding protein cDNAs and MAbs reveal subtypes of this branch of the ligand-gated ion channel gene superfamily, Neuron 5, 35-48.

Schulz et al. (1998), Dα3, a new functional α-subunit of nicotinic acetylcholine receptors from Drosophila, J. Neurochem. 71, 853-862

Sgard et al. (1998), Cloning and Functional Characterizsation of Two Novel Nicotinic Acetylcholine Receptor α subunits form the Insect Pest Myzus persicae; J. Neurochem 71, 903-912

## Patentansprüche

1. Nukleinsäure umfassend eine Sequenz ausgewählt aus
(a) der Sequenz gemäß SEQ ID NO: 1,
(b) zumindest 14 Basenpaare langen Teilsequenzen der unter (a) definierten Sequenz,
(c) Sequenzen, welche an die unter (a) definierte Sequenz hybridisieren,
(d) Sequenzen, welche eine zumindest 70 %ige Identität mit der Sequenz zwischen Position 43 und Position 1368 der unter (a) definierten Sequenz aufweisen,
(e) Sequenzen, welche zu der unter (a) definierten Sequenz komplementär sind und
(f) Sequenzen, welche aufgrund der Degeneriertheit des genetischen Codes für dieselbe Aminosäuresequenz kodieren wie die unter (a) bis (d) definierten Sequenzen.

2. Vektor umfassend zumindest eine Nukleinsäure gemäß Anspruch 1.

3. Vektor nach Anspruch 2, dadurch gekennzeichnet, dass das Nukleinsäuremolekül funktionell mit regulatorischen Sequenzen verknüpft ist, die die Expression der Nukleinsäure in pro- oder eukaryotischen Zellen gewährleisten.

4. Wirtszelle enthaltend eine Nukleinsäure gemäß Anspruch 1 oder einen Vektor gemäß Anspruch 2 oder 3.

5. Wirtszelle nach Anspruch 4, dadurch gekennzeichnet, dass es sich um eine pro- oder eukaryotische Zelle handelt.

6. Wirtszelle nach Anspruch 5, dadurch gekennzeichnet, dass die prokaryotische Zelle E.coli ist.

7. Wirtszelle nach Anspruch 5, dadurch gekennzeichnet, dass die eukaryotische Zelle eine Säuger- oder Insektenzelle ist.

8. Polypeptid, welches von einer Nukleinsäure gemäß Anspruch 1 kodiert wird.

9. Polypeptid, welches die biologische Funktion einer Acetylcholinrezeptor-β-Untereinheit ausübt und eine Aminosäuresequenz umfasst, die eine zumindest 40%ige Identität mit der Sequenz gemäß SEQ ID NO: 2 aufweist.

10. Acetylcholinrezeptor umfassend zumindest ein Polypeptid gemäß Anspruch 8 oder 9.

11. Verfahren zum Herstellen eines Polypeptids gemäß Anspruch 8 oder 9 umfassend
(a) das Kultivieren einer Wirtszelle gemäß einem der Ansprüche 4 bis 7 unter Bedingungen, die die Expression der Nukleinsäure gemäß Anspruch 1 gewährleisten, und
(b) die Gewinnung des Polypeptids aus der Zelle oder dem Kulturmedium.

12. Antikörper, welcher spezifisch mit dem Polypeptid gemäß Anspruch 8 oder 9, oder mit dem Rezeptor gemäß Anspruch 10 reagiert.

13. Transgener Invertebrat enthaltend eine Nukleinsäure gemäß Anspruch 1.

14. Transgener Invertebrat nach Anspruch 13, dadurch gekennzeichnet, dass es sich um Drosophila melanogaster oder Caenorhabditis elegans handelt.

15. Verfahren zum Herstellen eines transgenen Invertebraten gemäß Anspruch 13 oder 14 umfassend das Einbringen einer Nukleinsäure gemäß Anspruch 1 oder eines Vektors gemäß Anspruch 2 oder 3.

16. Transgene Nachkommen eines Invertebraten gemäß Anspruch 13 oder 14.

17. Verfahren zum Herstellen einer Nukleinsäure gemäß Anspruch 1 umfassend die folgenden Schritte:
(a) Vollständige chemische Synthese auf an sich bekannte Weise oder
(b) chemische Synthese von Oligonukleotiden, Markieren der Oligonukleotide, Hybridisieren der Oligonukleotide an DNA einer Insekten-cDNA-Bank, Selektieren von positiven Klonen und Isolieren der hybridisierenden DNA aus positiven Klonen oder
(c) chemische Synthese von Oligonukleotiden und Amplifizierung der Ziel-DNA mittels PCR.

18. Regulatorische Region, welche natürlicherweise die Transkription einer Nukleinsäure gemäß Anspruch 1 in Insektenzellen kontrolliert und eine spezifische Expression gewährleistet.

19. Verfahren zum Auffinden neuer Wirkstoffe für den Pflanzenschutz oder von pharmazeutischen Wirkstoffen für die Behandlung von Mensch oder Tier, insbesondere von Verbindungen, welche die Leitungseigenschaften von Rezeptoren gemäß Anspruch 10 verändern, umfassend die folgenden Schritte:
(a) Bereitstellen einer Wirtszelle gemäß einem der Ansprüche 4 bis 7,
(b) Kultivieren der Wirtszelle in der Gegenwart einer Verbindung oder eines Gemisches von Verbindungen, und
(c) Detektieren veränderter Leitungseigenschaften.

20. Verfahren zum Auffinden einer Verbindung, die an Rezeptoren gemäß Anspruch 10 bindet, umfassend die folgenden Schritte:
(a) Inkontaktbringen einer Wirtszelle gemäß einem der Ansprüche 4 bis 7, eines Polypeptids gemäß Anspruch 8 oder 9, oder eines Rezeptors gemäß Anspruch 10 mit einer Verbindung oder einem Gemisch von Verbindungen unter Bedingungen, die die Interaktion zumindest einer Verbindung mit der Wirtszelle, dem Polypeptid oder dem Rezeptor erlauben, und
(b) Bestimmen der Verbindung(en), die spezifisch an die Rezeptoren binden.

21. Verfahren zum Auffinden von Verbindungen, die die Expression von Rezeptoren gemäß Anspruch 10 verändern, umfassend die folgenden Schritte:
(a) Inkontaktbringen einer Wirtszelle gemäß einem der Ansprüche 4 bis 7 oder eines transgenen Invertebraten gemäß Anspruch 13 oder 14 mit einer Verbindung oder einem Gemisch von Verbindungen,
(b) Bestimmen der Rezeptorkonzentration, und
(c) Bestimmen der Verbindung(en), die die Expression des Rezeptors spezifisch beeinflussen.

22. Verwendung einer Nukleinsäure gemäß Anspruch 1, eines Vektors gemäß Anspruch 2 oder 3, einer Wirtszelle gemäß einem der Ansprüche 4 bis 7, eines Polypeptids gemäß Anspruch 8 oder 9, eines Acetylcholinrezeptors gemäß Anspruch 10, eines Antikörpers gemäß Anspruch 12, eines transgenen Invertebraten gemäß Anspruch 13 oder 14 oder einer regulatorischen Region gemäß Anspruch 18 zum Auffinden neuer Wirkstoffe für den Pflanzenschutz oder von pharmazeutischen Wirkstoffen für die Behandlung von Mensch oder Tier.
